# EUROPEAN PATENT APPLICATION

(11) **EP 4 209 594 A1**
(43) Date of publication of application: **12.07.2023**
(21) Application number: 21952112.7
(22) Date of filing: 06.12.2021
(51) Int. Cl.: C12N 15/866, C12N 5/10, C12N 15/35, C07K 14/015

(54) **EXPRESSION CASSETTE OF GENE CONTAINING OVERLAPPED OPEN READING FRAME AND APPLICATION OF EXPRESSION CASSETTE IN INSECT CELLS**

(30) Priority: 26.11.2021 CN 202111425804
(71) Applicant: Genevoyager (Wuhan) Co., Ltd., Wuhan, Hubei 430000 (CN)
(72) Inventor: XIAO, He, Wuhan, Hubei 430000 (CN); HE, Xiaobin, Wuhan, Hubei 430000 (CN); HUANG, Gang, Wuhan, Hubei 430000 (CN); HU, Ying, Wuhan, Hubei 430000 (CN); DU, Liang, Wuhan, Hubei 430000 (CN); WANG, Mengdie, Wuhan, Hubei 430000 (CN)
(74) Representative: Bayramoglu et al.
(86) International application number: PCT/CN2021/135642
(87) International publication number: WO 2023/092643

(57) **Abstract**

The disclosure belongs to the field of genetic engineering and discloses an expression cassette of a gene including overlapping open reading frames and an application thereof in an insect cell. The expression cassette includes from 5' to 3' and operably linked: a promoter capable of driving transcription in the insect cell; an artificially constructed sequence; overlapping open reading frames missing only a first translation start codon; wherein the artificially constructed sequence includes a first intron and a second intron with splicing activity in the insect cell, the first intron and the second intron share a 5' part or a 3' part, and the artificially constructed sequence does not include a translation start codon; during a post-transcriptional processing process, through an alternative splicing function of the first intron and the second intron, a translation start codon AUG can be formed. The disclosure uses the intron alternative splicing function to regulate relative expression of different capsid proteins and different Rep proteins, which can produce rAAV with high packaging rate and infectivity on a large scale in the insect cell.

## Description

### Technical Field

The disclosure belongs to the technical field of genetic engineering, and more particularly relates to an expression cassette of a gene including overlapping open reading frames and an application thereof in an insect cell.

### Description of Related Art

The gene including partially or completely overlapping open reading frames (ORF) has been found in mammalian genomes, and the overlapping open reading frames are particularly common in viral genomes. The regulation of expression of overlapping genes including the overlapping open reading frames is much more complicated than non-overlapping genes, and often requires an intron or multiple promoters for regulation.

The adeno-associated virus (AAV), also known as adeno-associated virus, belongs to the Parvoviridae family in the Dependovirus genus, is the simplest single-stranded DNA-deficient virus found so far, and requires a helper virus (usually adenovirus) to participate in replication. The recombinant adeno-associated virus (rAAV) is currently one of the most promising vectors in the field of gene therapy due to characteristics such as wide host range, low immunogenicity, high safety, and long-term stable expression of exogenous genes in animals. With the approval of the first recombinant adeno-associated virus (rAAV)-mediated gene therapy drug, the demand for large-scale manufacturing technology of AAV vectors is increasing (Yla-Herttuala S., 2012, Mol Ther, 20: 1831-1832).

The cap gene in the adeno-associated virus genome encodes a viral VP capsid protein, which includes 3 overlapping open reading frames, respectively encoding three types of subunits, VP1, VP2, and VP3. The stoichiometric ratio of VP1, VP2, and VP3 in AAV from a wild-type virus is approximately 1:1:10. The rep gene encodes four overlapping multifunctional proteins Rep78, Rep68, Rep52, and Rep40, which mainly participate in the replication of AAV. In a mammalian cell, the normal stoichiometric ratio expression of the three AAV capsid proteins may be regulated by the alternate use of two splice acceptor sequences of the intron and the use of ACG suboptimal start codon for VP2, thereby obtaining the normal recombinant adeno-associated virus by packaging. However, due to differences in intron splicing mechanisms in the mammalian cell and the insect cell, expression strategies that emerge in the mammalian cell do not replicate in the insect cell, thereby resulting in the inability of wild-type AAV to package into a suitable capsid in the insect cell.

In order to overcome the above issue, Urabe et al. developed an insect cell production system by replacing the start codon AUG of VP1 with the suboptimal start codon ACG to construct a single polycistronic mRNA. The polycistronic mRNA may express all three AAV2 VP proteins without splicing (Urabe et al., 2002, Hum Gene Ther, 13: 1935-1943). However, the serotypes of AAV are numerous and increasing day by day, and the transformation method by Urabe et al. is not applicable to all serotypes. For example, AAV5 particles produced in a baculovirus system using ACG as the start codon of the VP1 capsid protein by Urabe et al. has poor infectivity. In the method provided by Chinese patent CN 106544325 A, a different suboptimal start codon CTG is used to enhance the expression of VP1. Although such design improves the infectivity of AAV5 particles, the method lacks flexibility in regulating the relative content of VP1/VP2/VP3. At the same time, the method also appears to lack flexibility necessary for adjusting a serotype-specific sequence. In the method provided by Chinese patent CN 101522903 A, an artificial intron including a polh promoter sequence is inserted into the open reading frame of VP1, and two promoters are used to respectively express VP1 and VP2/VP3. Although such design can also implement the expression of VP1/VP2 /VP3 in the same reading frame, the method also cannot effectively regulate the relative amount of VP1/VP2/VP3, and the relative expression level of VP1/VP2/VP3 varies greatly in different serotypes, resulting in low production efficiency.

In an AAV insect cell production system, Urabe et al. constructed two independent expression cassettes, Rep78 and Rep52, which are inserted into the same baculovirus vector. Since the low expression of Rep78 is beneficial to the packaging of AAV, Rep52 uses a polh promoter, and Rep78 uses an △IE1 promoter that is less active than the polh promoter (Urabe et al., 2002, Hum Gene Ther, 13: 1935-1943). However, some studies have found that the AAV Rep protein expression method developed by Urabe et al. has the issue of unstable baculovirus vector passage. In order to solve the issue, Chinese patent CN 103849629 A discloses a method for producing AAV in an insect cell, which replaces the translation start codon of AAV Rep78 protein with ACG. Although such method can implement the expression of Rep78 and Rep52 proteins in the same reading frame, the method lacks the regulating function on the relative expression of the Rep78 and Rep52 proteins.

Therefore, it is very necessary to develop means and methods for the stable and large-scale production of the recombinant adeno-associated virus in the insect cell.

### SUMMARY

In view of the defects of the prior art, the objective of the disclosure is to provide an expression cassette of a gene including overlapping open reading frames and an application thereof in an insect cell, which can implement stable and large-scale production of an recombinant adeno-associated virus with high packaging rate and infectious activity in the insect cell, and is applicable to various serotypes.

In order to achieve the above objective, the disclosure provides an expression cassette for expressing a gene including overlapping open reading frames in an insect cell, which includes from 5' to 3' and operably linked:
a promoter capable of driving transcription in the insect cell;
an artificially constructed sequence;
overlapping open reading frames missing only a first translation start codon;
wherein the artificially constructed sequence includes a native or engineered first intron and second intron with splicing activity in the insect cell, the first intron and the second intron share a 5' part or a 3' part, and the artificially constructed sequence does not include a translation start codon;
during a post-transcriptional processing process, through an alternative splicing function of the first intron and the second intron, a translation start codon AUG can be formed in the artificially constructed sequence, thereby implementing the regulation of translation and expression of different protein encoding genes in the overlapping open reading frames.

Preferably, the first intron or the second intron is located between any two adjacent nucleotides in ATG.

Preferably, the artificially constructed sequence includes from 5' to 3' and operably linked: a 5' part of the first intron, an adenine nucleotide (A), a 5' part of the second intron, the shared 3' part, a thymine nucleotide (T), and a guanine nucleotide (G);
or the artificially constructed sequence includes from 5' to 3' and operably linked: a 5' part of the first intron, an adenine nucleotide (A), a thymine nucleotide (T), a 5' part of the second intron, the shared 3' part, and a guanine nucleotide (G).

Preferably, 5'-terminus nucleotides of the first intron and the second intron are both GTNN, and 3'-terminus nucleotides of the first intron and the second intron are both NNAG, wherein N is any one of the four nucleotides, A, T, C, and G.

Preferably, the gene including the overlapping open reading frames is a cap gene of AAV or a rep gene of AAV.

Preferably, the promoter is a polh promoter or a p10 promoter.

Preferably, the gene including the overlapping open reading frames is the cap gene of AAV, and the promoter is the p10 promoter.

Preferably, the gene including the overlapping open reading frames is the rep gene of AAV, and the promoter is the polh promoter.

According to another aspect of the disclosure, there is provided a nucleic acid molecule, which includes a first expression cassette. The first expression cassette is the expression cassette.

Preferably, the nucleic acid molecule further includes a second expression cassette. The second expression cassette is the expression cassette, and a gene expressed by the second expression cassette is different from a gene expressed by the first expression cassette.

Preferably, the second expression cassette is in an antisense orientation with respect to the first expression cassette.

Preferably, the second expression cassette is in a sense orientation with respect to the first expression cassette.

Preferably, the nucleic acid molecule further includes an exogenous gene and an AAV inverted terminal repeat sequence located at two terminals of the exogenous gene.

Preferably, the exogenous gene is a reporter gene. The reporter gene is at least one of a chloramphenicol acetyltransferase encoding gene, a β-galactosidase encoding gene, a β-glucuronidase encoding gene, a Renilla luciferase encoding gene, an alkaline phosphatase encoding gene, a firefly luciferase encoding gene, a green fluorescent protein encoding gene, and a red fluorescent protein encoding gene.

Preferably, the exogenous gene is a gene encoding a drug polypeptide or a gene encoding an antigenic protein for preparing a veterinary or human vaccine.

According to another aspect of the disclosure, there is provided a vector, which includes the expression cassette.

Preferably, the vector is an insect cytocompatible vector.

Preferably, the vector is at least one of a plasmid and a virus.

According to another aspect of the disclosure, there is provided an application of the vector for preparing a recombinant adeno-associated virus in an insect cell.

According to another aspect of the disclosure, there is provided an application of the vector for preparing an AAV capsid in vitro, and the gene including the overlapping open reading frames is the cap gene of AAV.

According to another aspect of the disclosure, there is provided an insect cell, which includes the expression cassette.

Preferably, the expression cassette is integrated into a genome of the insect cell.

Preferably, the insect cell is a Spodoptera frugiperda cell, a Trichoderma cell, a Drosophila cell, or a mosquito cell.

According to another aspect of the disclosure, there is provided a cell culture, which includes the insect cell and a culture medium.

Preferably, the culture medium includes an AAV genome.

According to another aspect of the disclosure, there is provided a recombinant adeno-associated virion, which is obtained by culturing the insect cell under conditions capable of producing the recombinant adeno-associated virion, and then recovering.

In general, compared with the prior art, the above technical solutions conceived by the disclosure have the following beneficial effects:
(1) The expression cassette of the disclosure may be used to express various polypeptides, such as the VP proteins (VP1, VP2, VP3) or the Rep proteins (Rep78, Rep52) of the recombinant adeno-associated virus) encoded by the gene including the overlapping open reading frames in the insect cell. The disclosure does not need to change the original translation start codon ATG of a VP1 protein encoding sequence or a Rep78 protein encoding sequence in wild-type AAV. Through inserting two introns downstream of a promoter and near the start codon ATG, and using the splicing function of the two introns to perform alternative splicing on the transcribed mRNA, two or more types of mRNA are obtained in appropriate ratios, which are respectively translated into the VP1, VP2/VP3 proteins or the Rep78 and Rep52 proteins.
(2) The disclosure uses the alternative splicing function of the two introns to implement the regulation strategy for overlapping gene expression, which can more effectively control the stoichiometric ratio of the VP1, VP2, and VP3 proteins and the relative expression of Rep78/Rep52 compared with the method using a suboptimal codon, so as to implement stable and large-scale production of the recombinant adeno-associated virus in the insect cell.
(3) The disclosure uses the intron alternative splicing strategy to implement the production of the recombinant adeno-associated virus in the insect cell, which is applicable to various AAV serotypes, so that the preparation of rAAV is more flexible and has a wider application range.
(4) In the disclosure, the cap gene and the rep gene of AAV and an ITR core expression element with the exogenous gene are constructed in the same recombinant baculovirus vector, and the vector is used to transfect the insect host cell, which can stably produce the recombinant AAV virion in high yield.

### BRIEF DESCRIPTION OF THE DRAWINGS

FIG. 1 is a schematic diagram of expression regulation of a cap gene and a rep gene in wild-type AAV of the disclosure.

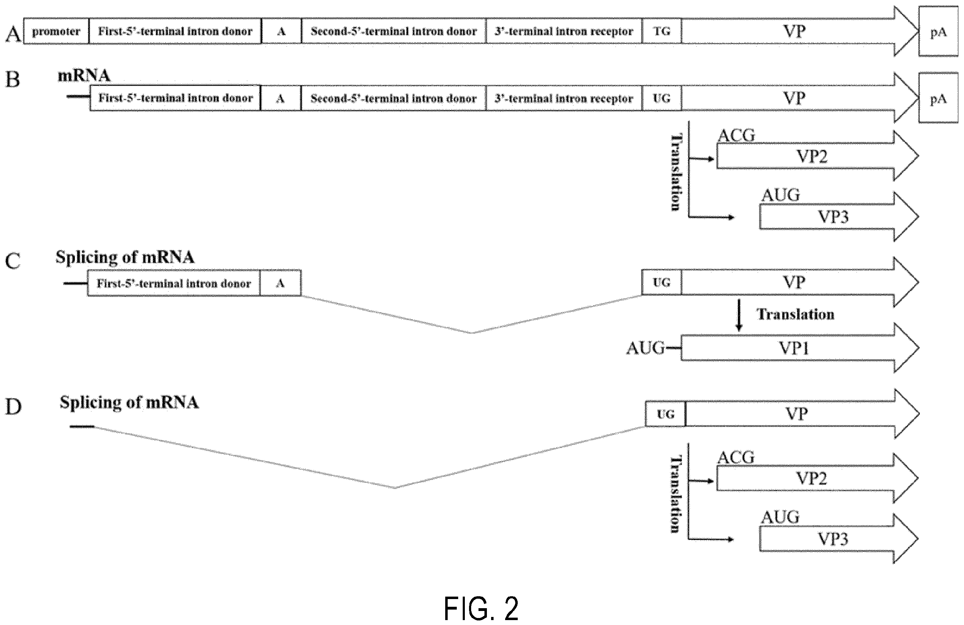

FIG. 2 is a schematic diagram of expression regulation of an expression cassette of a cap gene in Example 1 of the disclosure, wherein content A is a schematic diagram of a DNA chain of the expression cassette of the cap gene; content B is a schematic diagram of translation and expression of VP2 and VP3 proteins when neither a first intron nor a second intron is spliced during a post-transcriptional processing process; content C is a schematic diagram of translation and expression of a VP1 protein after the second intron is spliced during the post-transcriptional processing process; content D is a schematic diagram of translation and expression of the VP2 and VP3 proteins after the first intron is spliced during the post-transcriptional processing process.

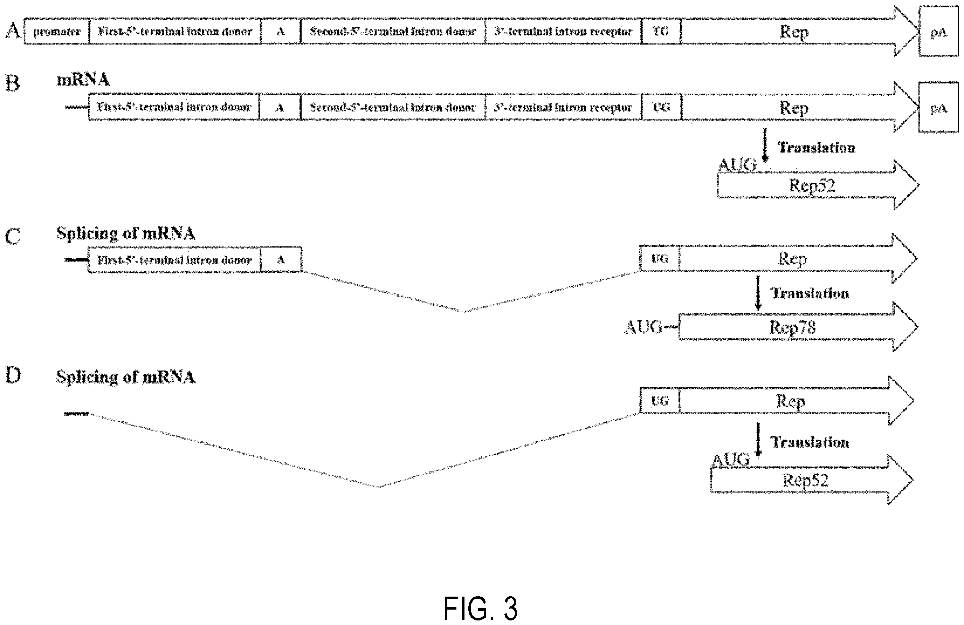

FIG. 3 is a schematic diagram of expression regulation of an expression cassette of a rep gene in Example 2 of the disclosure, wherein content A is a schematic diagram of a DNA chain of the expression cassette of the rep gene; content B is a schematic diagram of translation and expression of a Rep52 protein when neither a first intron nor a second intron is spliced during a post-transcriptional processing process; content C is a schematic diagram of translation and expression of a Rep78 protein after the second intron is spliced during the post-transcriptional processing process; content D is a schematic representation of translation and expression of the Rep52 protein after the first intron is spliced during the post-transcriptional processing process.
FIG. 4 is a western blot detection diagram of a recombinant baculovirus vector Ac1 including an expression cassette of a cap gene in Example 3 of the disclosure and a recombinant baculovirus vector Ac0 expressing a VP protein prepared in Comparative Example.
FIG. 5 is a western blot detection diagram of a recombinant baculovirus vector Ac2 including an expression cassette of a rep gene expressing a Rep protein in Example 3 of the disclosure.
FIG. 6 is a silver-stained image of a purified recombinant AAV virion subjected to SDS-PAGE after transfecting a host cell with recombinant AAV bacmids CRI-0 and CRI-1 in Example 6 of the disclosure and shows three capsid proteins VP1/VP2/VP3.
FIG. 7 are effect diagrams of a purified recombinant AAV virion infecting a 293T cell after transfecting a host cell with recombinant AAV bacmids CRI-0 and CRI-1 in Example 6 of the disclosure.
FIG. 8 are silver-stained images of a purified recombinant AAV virion subj ected to SDS-PAGE after transfecting a host cell with recombinant AAV bacmids CRI-2 (A), CRI-3 (B), and CRI-4 (C) in Example 7 of the disclosure and shows three capsid proteins VP1/VP2/VP3.
FIG. 9 are effect diagrams of a purified recombinant AAV virion infecting a 293T cell after transfecting a host cell with recombinant AAV bacmids CRI-2, CRI-3, and CRI-4 in Example 7 of the disclosure.

### DETAILED DESCRIPTION OF DISCLOSED EMBODIMENTS

In order for the objectives, technical solutions, and advantages of the disclosure to be clearer, the disclosure will be further described in detail below with reference to the drawings and the embodiments. It should be understood that the specific embodiments described herein are only used to explain the disclosure, but not to limit the disclosure.

### Terminology Explanation

As used herein, the term "operably linked" refers to the linkage of polynucleotide (or polypeptide) sequences in a functional relationship. Two nucleotide sequences are "operably linked" when the two nucleotide sequences are placed in a functional relationship. For example, a transcriptional regulatory sequence (for example, a promoter) is operably linked to a gene encoding sequence if the transcriptional regulatory sequence affects the transcription of the gene encoding sequence.

The term "expression cassette" refers to a nucleic acid construct including an operably linked encoding sequence and regulatory sequence when introduced into a host cell, which respectively cause transcription and/or translation of an RNA or a polypeptide. The expression cassette is understood to include a promoter that allows transcription to start, an open reading frame for a target gene, and a transcription terminator. Typically, a promoter sequence is placed upstream of the target gene at a distance compatible with expression control of the target gene.

The term "open reading frame" (ORF) is a normal nucleotide sequence of a structural gene that has the potential to encode a protein or a polypeptide and has no stop codon to interrupt translation starting with a start codon and ending with a stop codon. On an mRNA chain, a ribosome starts to translate from the start codon, synthesizes a polypeptide chain along a mRNA sequence and continues until the stop codon is encountered, and the elongation reaction of the polypeptide chain is terminated.

The term "vector" refers to a nucleic acid molecule, such as a plasmid vector, a cosmid vector, an artificial chromosome, a phage vector, and other viral vectors designed to transport, transfer, and/or store a genetic material, and express the genetic material and/or integrate the genetic material into a chromosomal DNA of a host cell. The vector usually consists of at least three basic units, that is, a replication origin, a selectable marker, and a multiple cloning site.

The term "intron", also known as a spacer sequence, refers to a segment without an encoding function in a gene or an mRNA molecule, which is an intervening sequence in a eukaryotic cell DNA. An intron sequence is transcribed in an mRNA precursor, removed by splicing, and ultimately absent from a mature mRNA molecule. According to whether the splicing process is spontaneous or processed by a spliceosome, the intron is divided into a self-splicing intron and a spliceosome intron. The self-splicing intron is a special type of intron and is a ribozyme that may be excised by its own function to leave the mRNA. The intron involved in the disclosure is the spliceosome intron. The splicing of such intron requires the help of a spliceosome. There are a splicing donor sequence and a splicing acceptor sequence at two terminals of the intron sequence, which are sequences on two sides of severing and rejoining sites. The spliceosome is a ribonucleoprotein complex dynamically composed of a small nuclear RNA (snRNA) and a protein factor. The spliceosome recognizes a splicing site of the mRNA precursor and catalyzes a splicing reaction, completely splices the intron, and then reconnects upstream and downstream RNA sequences.

The term "AAV serotype": since the discovery of AAV, more than 100 AAV serotypes or mutants have been isolated from adenoviruses, humans, primates, and some mammals, of which AAV1-9 is mainly applied. The main difference between rAAV vectors of different serotypes is differences in capsid proteins. Different AAV serotypes have certain differences in infection efficiency and tissue specificity.

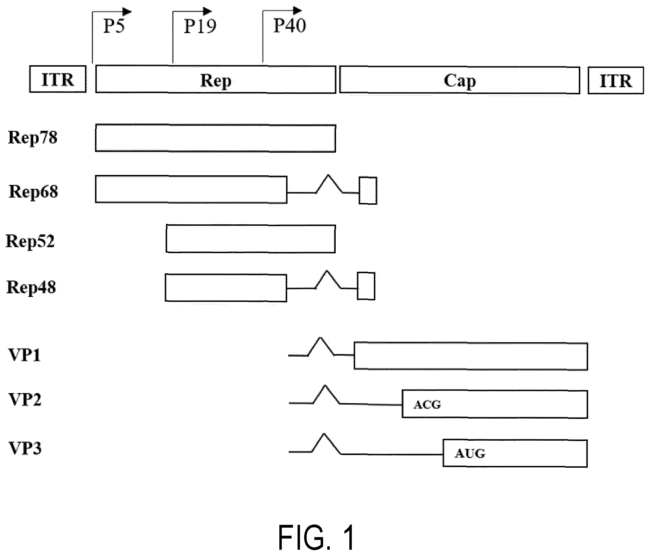

The genome structures of all known adeno-associated virus serotypes are very similar. AAV is a single-stranded DNA virus with a simple genome structure and a full length of about 4.7 kb. As shown in FIG. 1, a genome includes an expression cassette of a rep gene, an expression cassette of a cap gene, and an AAV inverted terminal repeat (ITR) sequence located at two terminals of the genome. ITR is a palindromic structure of 125 nucleotides at the two terminals of the genome, which can form a self-complementary inverted T hairpin structure and is a cis-acting element required for the initiation of DNA replication and the packaging of a recombinant AAV genome into an infectious virion. As a defective virus, AAV cannot replicate independently in the absence of a helper virus, so AAV can only be integrated into a chromosome of a host cell at a specific point and is in a latent state. In the presence of the helper virus, the increased expression level of the rep gene can rescue the AAV genome integrated in the chromosome of the host cell, which is massively replicated to obtain AAV DNA. A single-stranded rAAV genome is packaged into the infectious virion under the function of a VP capsid protein. The cap gene encodes a structural VP capsid protein, which includes 3 overlapping open reading frames, respectively encoding three types of subunits, VP1, VP2, and VP3. VP1, VP2, and VP3 include different start codons and share one stop codon, and VP1 and VP2 share a VP3 sequence. An N terminus of VP1 has a conserved phospholipase A2 sequence. The sequence is related to the escape of a virus from a body and is critical for infectivity. The VP2 protein is not essential for assembly or infection. A core of the VP3 protein consists of a conserved β-barrel motif, which determines differences in receptors that different serotypes of AAV interact with the host cell. The correct ratio of the three proteins in wild-type AAV is 3:3:54, which is approximately 1:1:10. The rep gene encodes four overlapping multifunctional proteins Rep78, Rep68, Rep52, and Rep40. The Rep78 and Rep68 proteins participate in the replication and integration of AAV and may be combined with a specific sequence in the ITR. The Rep52 and Rep40 proteins have helicase and ATPase activities, and participate in the assembly of a virus while participating in the replication of a single-stranded genome. Whether in a mammalian cell or an insect cell, unspliced mRNA encoding the Rep78 and Rep52 proteins is sufficient for rAAV production.

The nucleic acid molecule mentioned in the disclosure may be a DNA molecule or an RNA molecule. It is known to persons skilled in the art that an RNA sequence is substantially similar to or has a certain degree of sequence identity with a DNA sequence, and thymine (T) in the DNA sequence may be considered equivalent to uracil (U) in the RNA sequence.

The intron mentioned in the disclosure may be a native intron or an engineered intron and has splicing activity in the insect cell, but the source is not limited to the insect cell.

An expression cassette for expressing a gene including overlapping open reading frames in an insect cell provided by the disclosure includes from 5' to 3' and operably linked:
a promoter capable of driving transcription in the insect cell;
an artificially constructed sequence;
overlapping open reading frames missing only a first translation start codon;
wherein the artificially constructed sequence includes a native or engineered first intron and second intron with splicing activity in the insect cell, the first intron and the second intron share a 5' part or a 3' part, and the artificially constructed sequence does not include a translation start codon;
during a post-transcriptional processing process, through an alternative splicing function of the first intron and the second intron, a translation start codon AUG can be formed in the artificially constructed sequence, thereby implementing the regulation of translation and expression of different protein encoding genes in the overlapping open reading frames.

In some embodiments, the first intron or the second intron is located between any two adjacent nucleotides in ATG. The arrangement order of the first intron and the second intron is not particularly limited. Taking the first intron being located upstream of the second intron as an example, when the first intron is located between any two adjacent nucleotides in ATG, and a part (a 3' part) in the second intron that is not shared with the first intron is located downstream of G in ATG, during the post-transcriptional processing process, through the splicing function of the first intron, the translation start codon AUG can be formed upstream of the overlapping open reading frames. When the second intron is located between any two adj acent nucleotides in ATG, and a part (a 5' part) in the first intron that is not shared with the second intron is located upstream of A in ATG, during the post-transcriptional processing process, through the splicing function of the second intron, the translation start codon AUG can be formed upstream of the overlapping open reading frames.

In some embodiments, using the intron alternative splicing function, an expression cassette including two introns may be used for correct expression of the AAV VP capsid protein or dominance expression of the Rep protein in the insect cell, wherein the overlapping open reading frames in the cap gene encode the AAV capsid proteins VP1, VP2, and VP3; and the overlapping open reading frames in the rep gene encode the Rep78 and Rep52 proteins.

In some embodiments, using the intron alternative splicing function, an expression cassette including two introns may be used to express the VP capsid proteins of various simian virus 40 (SV40) in the insect cell. SV40 is a double-stranded DNA virus with a 5.2 kb covalently closed circular genome, which also includes three capsid proteins VP1, VP2, and VP3. The VP3 protein is a truncated form of the VP2 protein, and VP3 and VP2 share a stop codon. A 5' part of a VP1 encoding sequence overlaps with a 3' part of VP2 and VP3 encoding sequences, but does not have the same open reading frame. In the mammalian cell, the expression of the VP proteins is regulated by an intron splicing mechanism. When the expression cassette of the disclosure is used to express the SV40 capsid protein in the insect cell, ORF that only lacks the first translation start codon in the expression cassette is the VP protein encoding sequence that only lacks the translation start codon ATG of the VP2 protein. The regulation of relative expression of the VP2 and VP3 proteins can be implemented by using the intron splicing strategy of the disclosure.

In some embodiments, the gene including the overlapping open reading frames is the cap gene of AAV. The overlapping open reading frame that only lacks the first translation start codon is the VP protein encoding sequence that only lacks the translation start codon ATG of the VP1 protein. The artificially constructed sequence includes from 5' to 3' and operably linked: a 5' part of the first intron, an adenine nucleotide (A), a 5' part of the second intron, the shared 3' part, a thymine nucleotide (T), and a guanine nucleotide (G); or a 5' part of the first intron, an adenine nucleotide, a thymine nucleotide, a 5' part of the second intron, the shared 3' part, and a guanine nucleotide.

The disclosure mutates an intron splicing site at an N terminus of a VP1 protein encoding sequence itself in wild-type AAV, so that a spliceosome in the insect cell cannot recognize the splicing site. The 5' part of the first intron and the shared 3' part form a complete first intron splicing site, and the 5' part of the second intron and the shared 3' part form a complete second intron splicing site, as shown in FIG. 2. During the post-transcriptional processing process, if the spliceosome in the insect cell recognizes the first intron splicing site and catalyzes a splicing reaction, a complete AUG-start site cannot be formed at the front end of the mRNA. When the ribosome recognizes the start codon of the VP2 protein from 5' to 3', the VP2 protein can be translated and expressed. Since the start codon of VP2 is a suboptimal codon ACG, which causes the leakage of ribosome scanning, the VP3 protein can be translated and expressed. If the spliceosome in the insect cell recognizes the second intron splicing site and catalyzes the splicing reaction, a complete AUG-start site is formed after splicing. The mRNA is translated from the first AUG, and the VP1 protein is expressed. If the spliceosome in the insect cell neither splices the first intron splicing site nor splices the second intron splicing site, there is no start codon at the 5'-terminus of the VP1 protein encoding sequence, and the VP2 and VP3 proteins are translated and expressed. The relative expression levels of the capsid proteins VP1, VP2, and VP3 are controlled through the splicing function with different probabilities of different introns.

In some embodiments, the gene including the overlapping open reading frames is the rep gene of AAV, and the overlapping open reading frame that only lacks the first translation start codon is a Rep protein encoding sequence that only lacks the translation start codon ATG of the Rep78 protein. The artificially constructed sequence includes from 5' to 3' and operably linked: a 5' part of the first intron, an adenine nucleotide, a 5' part of the second intron, the shared 3' part, a thymine nucleotide, and a guanine nucleotide; or a 5' part of the first intron, an adenine nucleotide, a thymine nucleotide, a 5' part of the second intron, the shared 3' part, and a guanine nucleotide.

The disclosure mutates one or more possible translation start sites upstream of the translation start codon of the Rep52 protein of a Rep78 protein encoding sequence in the wild-type AAV, so that the ribosome in the insect cell cannot recognize the sites. The 5' part of the first intron and the shared 3' part form a complete first intron splicing site, and the 5' part of the second intron and the shared 3' part form a complete second intron splicing site, as shown in, as shown in FIG. 3. During the post-transcriptional processing process, if the spliceosome in the insect cell recognizes the first intron splicing site and catalyzes the splicing reaction, a complete AUG-start site cannot be formed at the front end of the mRNA. When the ribosome recognizes the start codon of the Rep52 protein from 5' to 3', the Rep52 protein is translated and expressed. If the spliceosome in the insect cell recognizes the second intron splicing site and catalyzes the splicing reaction, a complete AUG-start site is formed after splicing. The mRNA is translated from the first AUG, and the Rep78 protein is expressed. If the spliceosome in the insect cell neither splices the first intron splicing site nor splices the second intron splicing site, there is no start codon at the 5'-terminus of the Rep78 protein encoding sequence, and the Rep52 protein is translated and expressed. The relative expression levels of the Rep78 protein and the Rep52 protein are controlled through the splicing function with different probabilities of different introns.

In some embodiments, preferably, the 5'-terminus nucleotides of the first intron and the second intron are both GTNN, and the 3'-terminus nucleotides of the first intron and the second intron are both NNAG, where N represents any one of the four nucleotides A, T, C, and G, which can regulate the translation and expression of the VP1, VP2, and VP3 proteins in a closer stoichiometric ratio, and is conducive to packaging into suitable capsids. Similarly, the translation and expression level of the Rep78 protein is lower than the Rep52 protein, which is beneficial to higher vector yield.

In some embodiments, the nucleotide sequence of the artificially constructed sequence is the sequence shown in SEQ ID No.2. As known to persons skilled in the art, the variant form of the intron sequence may be used in the technical solution of the disclosure. In certain structures, a sequence that is similar to the intron nucleotide sequence of the disclosure may be used, such as a sequence with the 5'-terminus of the 5' part of the first intron having at least 40%, 50%, 60%, 70%, 80%, or 90% identity with the sequence GTAAGTATTC, a sequence with the 5'-terminus of the 5' part of the second intron having at least 40%, 50%, 60%, 70%, 80% or 90% identity with the sequence GTAAGTATCG, and a sequence with the 3'-terminus of the 3' part shared by the first intron and the second intron having at least 40%, 50%, 60%, 70%, 80%, or 90% identity with the sequence AAACATTATTTATTTTGCAG.

In some embodiments, the VP capsid protein encoded by the cap gene may be the capsid protein of any AAV serotype, such as AAV serotype 2, AAV serotype 5, AAV serotype 8, and AAV serotype 9. The intron sequence and the splicing strategy involved in the disclosure are applicable to the correct expression of the VP capsid proteins of the existing various AAV serotypes.

In some embodiments, the promoter capable of driving transcription of the cap gene and the rep gene in the insect cell may be a polh promoter or a p 10 promoter. Preferably, the expression cassette of the cap gene adopts the p10 promoter, and the expression cassette of the rep gene adopts the polh promoter.

The disclosure also provides a nucleic acid molecule including the expression cassette of the cap gene and/or the expression cassette of the rep gene. The nucleic acid may include expression cassettes arranged in tandem, that is, in the same polarity or in an antisense orientation. Therefore, the expression cassette of the cap gene may be in an antisense orientation with respect to the expression cassette of the rep gene or may be in a sense orientation with respect to the expression cassette of the rep gene.

The disclosure also provides a recombinant baculovirus vector, preferably an insect cell-compatible vector, which includes the expression cassette of the cap gene and the expression cassette of the rep gene. The expression cassette of the cap gene is preferably the expression cassette constructed by the disclosure, and the expression cassette of the rep gene is preferably the gene expression cassette constructed by the disclosure. It should be understood that the rAAV vector also includes an exogenous gene and an AAV inverted terminal repeat sequence located at two terminals of the exogenous gene. ITR acts on the replication packaging and the site-directed integration of AAV, helping the vector to form a stable cyclic polymer and chromatin-like structure in the host cell for a long time, and the exogenous gene can also be continuously and stably expressed. The exogenous gene may be at least one nucleotide sequence encoding a gene of interest (GOI) product. The gene of interest product may be a therapeutic gene product, which is specifically a polypeptide, an RNA molecule (siRNA), or other gene products such as but not limited to lipoprotein esterase, apolipoprotein, cytokine, interleukin, or interferon; may also be an antigenic protein for preparing veterinary or human vaccines, such as the hepatitis B surface antigen, the rabies virus glycoprotein, and the capsid protein of the foot-and-mouth disease virus; or may also be a reporter protein to assess vector transformation and expression, such as but not limited to a fluorescent protein (a green fluorescent protein GFP, a red fluorescent protein RFP), chloramphenicol acetyltransferase, β-galactosidase, β-glucuronidase, Renilla luciferase, firefly luciferase, or alkaline phosphatase.

On the other hand, the disclosure also provides an application of the recombinant baculovirus vector for preparing a recombinant adeno-associated virus in an insect cell. The introduction of the vector into the insect cell can implement the expression of the VP1, VP2, and VP3 proteins in the correct stoichiometric ratio of 1:1:10, thereby packaging to produce the recombinant adeno-associated virus, which has a high packaging rate, and most of the known AAV serotypes (for example, AAV2, AAV5, AAV8, and AAV9) all have better infectivity. At the same time, the expression levels of the Rep78 and Rep52 proteins are controlled within a reasonable range, so that the AAV vector can be stably and massively produced in the insect cell. Also, the recombinant baculovirus vector including the expression cassette of the cap genes may be used to prepare AAV capsid in vitro.

On the other hand, the disclosure also provides an insect cell. The insect cell includes the expression cassette of the cap gene and/or the rep gene of AAV and may also include the expression cassette of the exogenous gene. At least one of the expression cassettes may be integrated into the genome of the host insect cell for stable expression, or the insect cell may transiently carry the nucleic acids including the expression cassettes.

In some embodiments, the insect cell may be any insect cell, such as but not limited to a Spodoptera frugiperda cell, a Trichoderma cell, a Drosophila cell, or a mosquito cell, preferably a Spodoptera frugiperda cell sf9.

In another aspect, the disclosure also provides a cell culture, which includes the insect cell and a culture medium.

In some embodiments, the culture medium includes an AAV genome.

On the other hand, the disclosure also provides a recombinant adeno-associated virion, which is obtained by culturing the insect cell under conditions capable of producing the recombinant adeno-associated virion, and then recovering.

The above technical solutions are described in detail below with reference to specific examples.

Example 1 Construction of a recombinant baculovirus vector including an expression cassette of a cap gene of AAV serotype 9
(1) Construction of the expression cassette of the cap gene: see FIG. 2, the expression cassette of the cap gene sequentially includes a p10 promoter, an artificially constructed sequence, and a nucleotide sequence encoding a VP protein of AAV serotype 9 that only lacks a translation start codon ATG of a VP1 protein from 5' to 3'. A nucleotide sequence of the p10 promoter is shown in SEQ ID No. 1. A nucleotide sequence of the artificially constructed sequence in the expression cassette of the cap gene of the example is shown in SEQ ID No. 2, which includes two intron splicing sites, wherein a nucleotide sequence of a first intron is shown in SEQ ID No.3, and a nucleotide sequence of a second intron is shown in SEQ ID No.4. The nucleotide sequence encoding the VP protein of AAV serotype 9 is shown in SEQ ID No. 5, and there is at least one nucleotide mutation at an amino terminus to eliminate possible splicing sites in the encoding sequence. The above sequences were connected through artificial direct synthesis or overlap extension PCR amplification to respectively obtain a construct 9K-1 whose nucleotide sequence is shown in SEQ ID No.6.
(2) Construction of the recombinant baculovirus vector: the construct 9K-1 was cloned into a pFastBac vector to prepare a transfer plasmid. The plasmid was transformed into a DH10Bac strain, and a recombinant baculovirus vector Ac1 was obtained through Tn7 transposition.

Example 2 Construction of a recombinant baculovirus vector including an expression cassette of a rep gene of AAV serotype 2
(1) Construction of the expression cassette of the rep gene: see FIG. 3, the expression cassette of the rep gene sequentially includes a polh promoter, an artificially constructed sequence, and a nucleotide sequence encoding a Rep protein of AAV serotype 2 that only lacks a translation start codon ATG of a Rep78 protein from 5' to 3'. A nucleotide sequence of a polh promoter is shown in SEQ ID No.7. A nucleotide sequence of the artificially constructed sequence in the expression cassette of the rep gene of the example is shown in SEQ ID No. 2, which includes two intron splicing sites, wherein a nucleotide sequence of a first intron is shown in SEQ ID No.3, and a nucleotide sequence of a second intron is shown in SEQ ID No.4. The nucleotide sequence encoding the Rep protein of AAV serotype 2 is shown in SEQ ID No. 8, which has multiple nucleotide mutations between a Rep78 translation start codon and a Rep52 translation start codon to eliminate possible translation start sites within the region. The above sequences are connected through artificial direct synthesis or overlap extension PCR amplification to obtain a construct 2R-1 whose nucleotide sequence is shown in SEQ ID No.9.
(2) Construction of a recombinant baculovirus vector: the same as Step (2) in Example 1. A recombinant baculovirus vector Ac2 including 2R-1 was obtained.

Comparative Example Construction of a recombinant baculovirus vector Ac0 and an expression cassette 2R-0 of a rep gene
(1) Construction of an expression cassette of a cap gene: in Comparative Example, the expression cassette of the cap gene was constructed with reference to the method of Urabe et al. (Urabe et al., 2002, Hum Gene Ther, 13: 1935-1943). A promoter of the expression cassette of the cap gene adopts a p10 promoter, the cap gene in the expression cassette encodes a VP protein of AAV serotype 9, and a translation start codon ATG of VP1 in the encoding sequence is mutated to a suboptimal codon ACG. The expression cassette of the cap gene is numbered 9K-0 whose nucleotide sequence is shown in SEQ ID No. 10.
(2) Construction of a recombinant baculovirus vector: the same as Step (2) in Example 1. A recombinant baculovirus vector Ac0 including 9K-0 was obtained.
(3) Construction of the expression cassette of the rep gene: the expression cassette of the rep gene was constructed with reference to the method of Chinese patent CN 103849629 A. A promoter of the expression cassette of the rep gene adopts a polh promoter. The rep gene in the expression cassette encodes a Rep protein of AAV serotype 2, and an original translation start codon ATG of a Rep78 protein of AAV was replaced with ACG. The expression cassette of the rep gene is numbered 2R-0.

### Example 3 Detection of expression of VP proteins (VP1, VP2, VP3) and Rep proteins (Rep78, Rep52)

The recombinant baculovirus vectors Ac1, Ac2, and Ac0 prepared in Example 1, Example 2, and Comparative Example were respectively transfected into a host cell line and cultured to obtain a recombinant baculovirus, and the expression of the VP proteins (VP1, VP2, VP3) and the Rep proteins (Rep78, Rep52) were respectively detected. The specific operation steps are as follows:
DNA of the recombinant baculovirus vector was extracted and transfected into a Sf9 insect cell to prepare a recombinant baculovirus BEV. The transfected Sf9 insect cell successfully produced BEV, and further infection of massively replicated BEV resulted in obvious cytopathic effect (CPE) in the Sf9 cells. Culture supernatant of the Sf9 cells with CPE was collected, which included a large amount of BEV, which was the 0th generation BEV (P0). At the same time, the Sf9 cells including a large amount of rAAV were collected. The obtained BEV-P0 infected the Sf9 cells in suspension culture at a multiplicity of infection (MOI=1). After infection for 72 h, the cell activity dropped to less than 50%. The cell culture medium was centrifuged at 1000 g for 5 min. The culture supernatant and the cell pellet were respectively collected. The supernatant was labeled as the 1st generation BEV-P1. Continue to expand the culture, and the obtained BEV-P1 infected the Sf9 cells in suspension culture at a multiplicity of infection (MOI=1). After infection for 72 h, the cell activity dropped to less than 50%. The cell culture medium was centrifuged at 1000 g for 5 min. The cell pellet was collected for western blot to examine the expression of the VP proteins (VP1, VP2, VP3) and the Rep proteins (Rep78, Rep52).

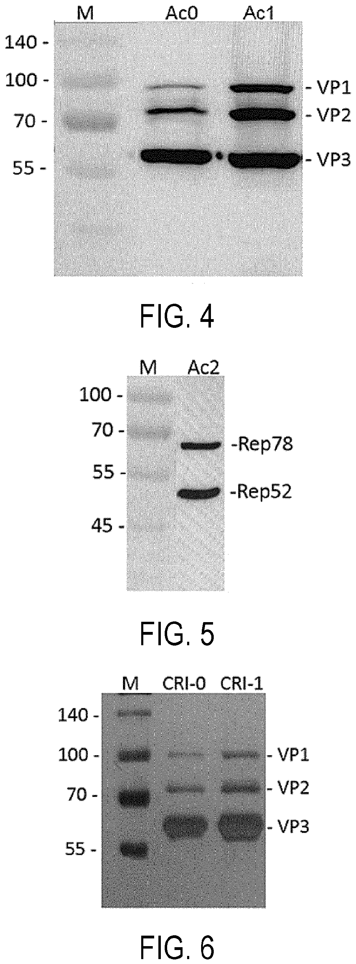

FIG. 4 is a western blot detection diagram of the VP proteins (VP1, VP2, and VP3) of the recombinant baculovirus vector Ac1 including the expression cassette of the cap gene and the control recombinant baculovirus vector Ac0. It can be seen from the figure that the above recombinant baculovirus vectors can both produce VP1, VP2, and VP3 in a relatively appropriate ratio.

FIG. 5 is a western blot detection diagram of the Rep proteins (Rep78 and Rep52) of the recombinant baculovirus vector Ac2 including the expression cassette of the rep gene. It can be seen from the figure that the recombinant baculovirus vector Ac2 can produce the Rep78 and Rep52 proteins, and the expression level of the Rep78 protein is lower than the Rep52 protein.

Example 4 Construction of a recombinant AAV bacmid for producing an AAV virus in an insect cell

With reference to Example 1 in the previous patent application CN 112553257 A of our company, the method for constructing the recombinant AAV bacmid for producing the AAV virus in the insect cell in the example includes the following steps:
(1) Construction of a homologous recombination vector including AAV essential functional elements, expression cassettes of cap and rep genes. Then, the essential functional elements in the homologous recombination vector were inserted into a C-terminus of an essential gene Ac135 (116492...117391) in a baculovirus genome through Red homologous recombination in Escherichia coli to obtain the recombinant baculovirus vector including the expression cassettes of the cap and rep genes, and numbered Bac-Cap-Rep.
(2) Construction of a shuttle vector including an ITR core element (ITR-GOI). The ITR core element is numbered IG-1 whose nucleotide sequence is shown in SEQ ID No.11. In the example, GOI in the ITR core element adopts an expression cassette of a red fluorescent protein mcherry gene, that is, the expression of mcherry is controlled by a miniEfla promoter, which is convenient for detecting the activity of rAAV. ITR and the expression cassette of the red fluorescent protein were constructed on the shuttle vector pFastDual.
(3) A competent cell including the Bac-Cap-Rep recombinant bacmid was transformed using the shuttle vector constructed in Step (2) above. ITR-GOI was inserted into a Tn7 site in the Bac-Cap-Rep recombinant bacmid using Tn7 recombination. Finally, a recombinant bacmid of a recombinant baculovirus genome including the functional protein components essential for producing rAAV and the ITR core element was obtained and numbered Bac-Cap-Rep-ITR-GOI.

The 2 different recombinant AAV bacmids constructed in the example both include the expression cassette of the cap gene, the expression cassette of the rep gene, and the ITR core element, as shown in Table 1.

**Table 1 List of constituent elements of 2 different recombinant AAV bacmids constructed in Example 4**

| Recombinant AAV bacmid (Bac-Cap-Rep-ITR-GOI) numbering | Functional protein components essential for producing rAAV and ITR core element | | |
|---|---|---|---|
| | Expression cassette of cap gene | Expression cassette of rep gene | ITR core element (ITR-GOI) |
| CRI-0 | 9K-0 | 2R-0 | I-G-1 |
| CRI-1 | 9K-1 | 2R-1 | I-G-1 |

### Example 5 Preparation of an AAV recombinant baculovirus

The recombinant AAV bacmids CRI-0 and CRI-1 prepared in Example 4 were respectively transfected into a host cell line and cultured to obtain an AAV recombinant baculovirus.

DNA of the recombinant bacmid was extracted and transfected into a Sf9 insect cell to prepare a recombinant baculovirus BEV and rAAV. The transfected Sf9 insect cell successfully produced BEV, and further infection of massively replicated and proliferated BEV resulted in obvious cytopathic effect (CPE) in the Sf9 cells. Culture supernatant of the Sf9 cells with CPE was collected, which included a large amount of BEV, which was the 0th generation BEV (P0). At the same time, the Sf9 cells including a large amount of rAAV were collected. The obtained BEV-P0 infected the Sf9 cells in suspension culture at a multiplicity of infection (MOI=1). After infection for 72 h, the cell activity dropped to less than 50%. The cell culture medium was centrifuged at 1000 g for 5 min. The culture supernatant and the cell pellet were respectively collected. The supernatant was labeled as the 1st generation BEV-P1, and the cells were labeled as rAAV packaged with BEV-P0.

### Example 6 Purification of a recombinant AAV virion and detection of packaging rate and virus titer thereof

Continue to expand the culture according to the operation of Example 5 until the Sf9 cells in suspension culture are infected with BEV-P2 seed virus according to a multiplicity of infection (MOI=1) for rAAV packaging, and the packaging volume is 300 mL to 400 mL. After infection for 3 days, the cell activity was monitored, and if the activity was less than 50%, the cell pellet and the supernatant were respectively harvested by centrifugation. The harvested cell pellet and supernatant were respectively purified. Nuclease (Benzonase) was added to the supernatant, treated in a water bath at 37°C for 60 min, and centrifuged at 5000 rpm for 10 min after treatment. The collected cell lysate and the collected supernatant were PEG precipitated, resuspended, and purified by iodixanol density gradient centrifugation (see Aslanidi et al., 2009, Proc. NatlAcad. Sci. USA, 206: 5059-5064 for the method). Finally, the purified virus was resuspended in 80 µL to 190 µL PBS, and 10 µL of the purified virus was run on SDS-PAGE gel for silver staining.

FIG. 6 provides a detection diagram of a purified recombinant AAV virion subjected to SDS-PAGE silver staining after transfecting a host cell with recombinant AAV bacmids CRI-0 and CRI-1. It can be seen from the result that the recombinant AAV bacmids constructed by the disclosure can implement VP1, VP2, and VP3 proteins in a relatively appropriate ratio in the insect cell through the regulation function of intron splicing, thereby packaging to form the virion.

In the example, Q-PCR was also adopted to detect the packaging rate of the harvested rAAV virus, and the detection of the rAAV packaging rate used a pair of primers (Q-ITR-F: GGAACCCCTAGTGATGGAGTT and Q-ITR-R: CGGCCTCAGTGAGCGA) targeting an ITR sequence. The detection of rAAV infection titer was performed by adopting the detection method in the previous patent application CN 112280801 A of our company. For the specific operation method, reference may be made to the method for plasmid-assisted rAAV infection titer detection in the patent. The detection result is shown in Table 2.

**Table 2 Detection result table of packaging rate and infection titer of rAAV virions produced using 2 different recombinant AAV bacmids**

| Recombinant AAV bacmid numbering | Cell packaging rate (VG/cell) | Virus infection titer (VG/mL) |
|---|---|---|
| CRI-0 | 4.75E+05 | 4.31E+08 |
| CRI-1 | 7.86E+05 | 5.37E+08 |

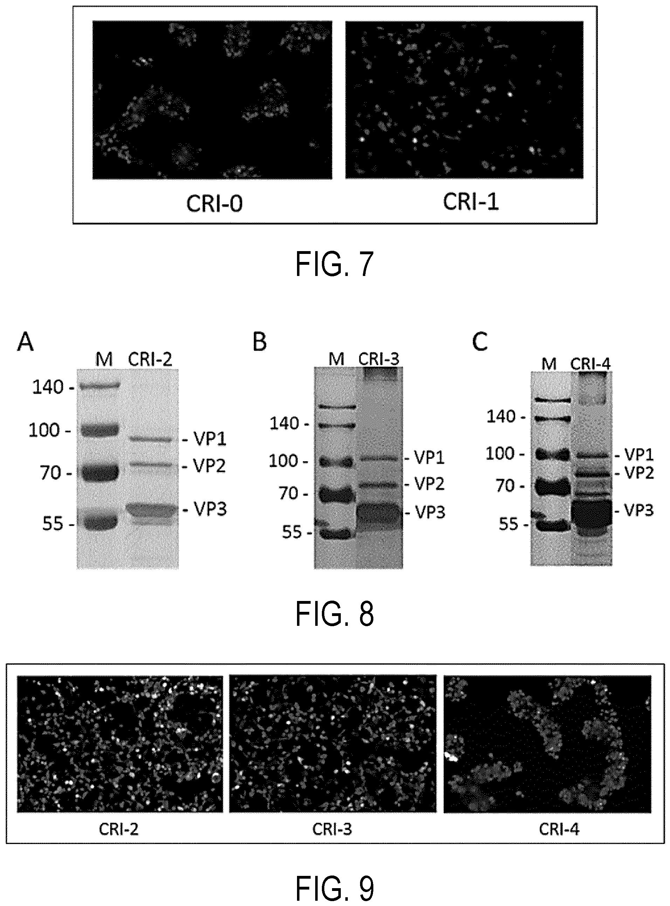

It can be seen from Table 2 and FIG. 7 that the recombinant AAV bacmid CRI-1 prepared by the disclosure can be used to transfect the insect cell to package to form the recombinant baculovirus, and the packaging rate and the virus infection titer are high.

### Example 7 Validation of intron regulation function in different AAV serotypes

In order to verify that the above intron regulation manner can work in different AAV serotypes, an expression cassette 2K-1 of a cap gene of AAV serotype 2, an expression cassette 5K-1 of a cap gene of AAV serotype 5, and an expression cassette 8K-1 of a cap gene of AAV serotype 8 were respectively constructed with reference to the method in Example 1. Nucleotide sequences of the constructs 2K-1, 5K-1, and 8K-1 are respectively shown in SEQ ID No. 12 to SEQ ID No. 14. An expression cassette of a rep gene in the example still adopts AAV serotype 2, because a Rep protein of AAV serotype 2 is suitable for the preparation of rAAV of various serotypes.

Referring to Example 4, 3 different recombinant AAV bacmids were constructed in the example, all of which included the expression cassette of the cap gene, the expression cassette of the rep gene, and the ITR core element, as shown in Table 3.

**Table 3 List of constituent elements of 3 different recombinant AAV bacmids constructed in Example 7**

| Recombinant AAV bacmid (Bac-Cap-Rep-ITR-GOI) numbering | Functional protein components essential for producing rAAV and ITR core element | | |
|---|---|---|---|
| | Expression cassette of cap gene | Expression cassette of rep gene | ITR core element (ITR-GOI) |
| CRI-2 | 2K-1 | 2R-1 | I-G-1 |
| CRI-3 | 5K-1 | 2R-1 | I-G-1 |
| CRI-4 | 8K-1 | 2R-1 | I-G-1 |

Referring to Example 5 and Example 6, in the example, the recombinant AAV bacmid was transfected into a host cell, and the purified recombinant AAV virion was detected by SDS-PAGE silver staining. The experimental result is shown in FIG. 8. The cell packaging rate and the virus infection titer of rAAV produced by the recombinant AAV bacmids of different serotypes were detected, and the detection result is shown in Table 4.

**Table 4 Detection result table of packaging rate and infection titer of rAAV virions produced using 3 different recombinant AAV bacmids in Example 7**

| Recombinant AAV bacmid numbering | Cell packaging rate (VG/cell) | Virus infection titer (VG/mL) |
|---|---|---|
| CRI-2 | 3.59E+05 | 3.89E+08 |
| CRI-3 | 3.88E+05 | 7.46E+08 |
| CRI-4 | 2.59E+05 | 9.28E+08 |

Combining the results of Table 4 and FIG. 9, it is shown that the disclosure regulates the expression of the VP1, VP2, and VP3 proteins in the insect cell in a relatively appropriate ratio through the intron splicing function, which is suitable for various AAV serotypes and can produce the rAAV virion on a large scale.

Persons skilled in the art can easily understand that the above are only preferred embodiments of the disclosure and are not intended to limit the disclosure. Any modifications, equivalent replacements, improvements, etc. made within the spirit and principle of the disclosure should be included within the protection scope of the disclosure.

## Claims

1. An expression cassette for expressing a gene comprising overlapping open reading frames in an insect cell, comprising from 5' to 3' and operably linked:
a promoter capable of driving transcription in the insect cell;
an artificially constructed sequence;
an overlapping open reading frame missing only a first translation start codon;
wherein the artificially constructed sequence comprises a native or engineered first intron and second intron with splicing activity in the insect cell, the first intron and the second intron share a 5' part or a 3' part, and the artificially constructed sequence does not comprise a translation start codon;
during a post-transcriptional processing process, through an alternative splicing function of the first intron and the second intron, a translation start codon AUG is formed in the artificially constructed sequence to regulate translation and expression of different protein encoding genes in the overlapping open reading frames.

2. The expression cassette according to claim 1, wherein the first intron or the second intron is located between any adjacent two nucleotides in ATG.

3. The expression cassette according to claim 2, wherein the artificial construction sequence comprises from 5' to 3' and operably linked: a 5' part of the first intron, an adenine nucleotide, a 5' part of the second intron, the shared 3' part, a thymine nucleotide, and a guanine nucleotide;
or the artificially constructed sequence comprises from 5' to 3' and operably linked: a 5' part of the first intron, an adenine nucleotide, a thymine nucleotide, a 5' part of the second intron, the shared 3' part, and a guanine nucleotide.

4. The expression cassette according to claim 1, wherein 5'-terminus nucleotides of the first intron and the second intron are both GTNN, and 3'-terminus nucleotides of the first intron and the second intron are NNAG, wherein N is any one of four nucleotides, A, T, C, and G.

5. The expression cassette according to claim 1, wherein the gene comprising the overlapping open reading frames is a cap gene of AAV or a rep gene of AAV.

6. The expression cassette according to claim 1, wherein the promoter is a polh promoter or a p10 promoter.

7. The expression cassette according to claim 6, wherein the gene comprising the overlapping open reading frames is a cap gene of AAV, and the promoter is the p10 promoter.

8. The expression cassette according to claim 6, wherein the gene comprising the overlapping open reading frames is a rep gene of AAV, and the promoter is the polh promoter.

9. A nucleic acid molecule, comprising: a first expression cassette, wherein the first expression cassette is the expression cassette according to any one of claims 1 to 8.

10. The nucleic acid molecule according to claim 9, further comprising: a second expression cassette, wherein the second expression cassette is the expression cassette according to any one of claims 1 to 8, and a gene expressed by the second expression cassette is different from a gene expressed by the first expression cassette.

11. The nucleic acid molecule according to claim 10, wherein the second expression cassette is in an antisense orientation with respect to the first expression cassette.

12. The nucleic acid molecule according to claim 10, wherein the second expression cassette is in a sense orientation with respect to the first expression cassette.

13. The nucleic acid molecule according to claim 9, further comprising: an exogenous gene and an AAV inverted terminal repeat sequence located at two terminals of the exogenous gene.

14. The nucleic acid molecule according to claim 13, wherein the exogenous gene is a reporter gene, and the reporter gene is at least one of a chloramphenicol acetyltransferase encoding gene, a β-galactosidase encoding gene, a β-glucuronidase encoding gene, a Renilla luciferase encoding gene, an alkaline phosphatase encoding gene, a firefly luciferase encoding gene, a green fluorescent protein encoding gene, and a red fluorescent protein encoding gene.

15. The nucleic acid molecule according to claim 13, wherein the exogenous gene is a gene encoding a drug polypeptide or a gene encoding an antigenic protein for preparing a veterinary or human vaccine.

16. A vector, comprising the expression cassette according to any one of claims 1 to 8.

17. The vector according to claim 16, wherein the vector is an insect cytocompatible vector.

18. The vector according to claim 16, wherein the vector is at least one of a plasmid and a virus.

19. An application of the vector according to claim 16 for preparing a recombinant adeno-associated virus in an insect cell.

20. An application of the vector according to claim 16 for preparing an AAV capsid in vitro, wherein a gene comprising the overlapping open reading frames is a cap gene of AAV.

21. An insect cell, comprising the expression cassette according to any one of claims 1 to 8.

22. The insect cell according to claim 21, wherein the expression cassette is integrated into a genome of the insect cell.

23. The insect cell according to claim 21, wherein the insect cell is a Spodoptera frugiperda cell, a Trichoderma cell, a Drosophila cell, or a mosquito cell.

24. A cell culture, comprising the insect cell according to claim 21 and a culture medium.

25. The cell culture according to claim 24, wherein the culture medium comprises an AAV genome.

26. A recombinant adeno-associated virion, obtained by culturing the insect cell according to claim 21 under conditions capable of producing the recombinant adeno-associated virion, and then recovering.
